# EUROPEAN PATENT APPLICATION

(11) **EP 3 006 915 A1**
(43) Date of publication of application: **13.04.2016**
(21) Application number: 14804477.9
(22) Date of filing: 26.05.2014
(51) Int. Cl.: G01N 1/28, G01N 1/22, G01N 30/00, G01N 30/88, G01N 33/497

(54) **SKIN GAS MEASUREMENT DEVICE AND SKIN GAS MEASUREMENT METHOD**

(30) Priority: 29.05.2013 JP 2013113392
(71) Applicant: NTT Docomo, Inc., Tokyo 100-6150 (JP); The University of Tokyo, Tokyo 113-8654 (JP)
(72) Inventor: YAMADA, Yuuki, Tokyo 100-6150 (JP); HIYAMA, Satoshi, Tokyo 100-6150 (JP); TAKEUCHI, Shoji, Tokyo 113-8654 (JP); OKUBO, Tatsuya, Tokyo 113-8654 (JP); ITABASHI Keiji, Tokyo 113-8654 (JP)
(74) Representative: Maury, Richard Philip
(86) International application number: PCT/JP2014/063788
(87) International publication number: WO 2014/192674

(57) **Abstract**

A skin gas measurement device includes a skin gas collecting unit that includes a skin gas collecting space having an opening that is to be attached to a skin surface, a porous material that is for adsorbing and concentrating a skin gas component that is emitted from the skin surface into the skin gas collecting space and that allows the adsorbed skin gas component to be desorbed at a relatively low temperature, and a heater for heating the porous material; and a skin gas measurement unit for measuring the skin gas component that is desorbed from the heated porous material.

## Description

### TECHNICAL FIELD

The present invention generally relates to measurement of gas (which is described as a "skin gas," hereinafter) components (which is described as "skin gas components," hereinafter) that are emitted from a skin surface of a living body, and specifically relates to a skin gas measurement device and a skin gas measurement method for measuring the skin gas components by collecting and concentrating the skin gas components.

### BACKGROUND ART

Recently, the national medical expenditure continues increasing, and "preventive medical care" is focused on that is for preventing occurrence and/or progress of a disease. In order for the preventive medical care to be successful, it may be desirable to implement a measurement device that can easily and simply examine and confirm own health conditions in detail anytime and anywhere, and a service (e.g., health advices) that takes into account individual differences. Thus far, it has been common to simply examine and confirm own health conditions by measuring a pulse, a blood pressure, a heart rate, an activity amount, a number of steps, and so forth. These are measurement devices that mainly use physical sensors, such as an acceleration sensor. However, there may be some bio information that may not be easily obtained only by the physical sensor. For example, in a medical institution or the like, biochemical and physiological data that may not be obtained only by the physical sensor is obtained by collecting and measuring a biological sample, such as blood, urine, lymph, and a cerebrospinal fluid, and it is used for examination and/or confirmation of health conditions and diagnosis of illness. However, these bio samples may not be suitable for easy and simple measurement because these bio samples may be technically difficult to collect for a typical person (a user) who is not a health care worker, may have some risks of infection during collection, or may involve invasion into a human body or a psychological burden.

A bio gas, such as an exhaled gas or a skin gas, can be considered as an example of one of bio samples which can be easily collected, for which there is no risk of infection, and which does not involve invasion into a human body or a psychological burden. Similar to a liquid sample, such as blood, a bio gas is a storehouse of biological information that reflects individual differences. It has been known that by measuring presence or absence, or concentrations of specific gas components in a bio gas, information on health conditions can be obtained. Among bio gases, a skin gas has a potential to accurately detect components of a bio gas and to accurately measure concentrations of the bio gas, compared to an exhaled gas. That is because, compared to an exhaled gas, active operation, such as blowing into a collecting device or the like, may not be required during collection and measurement, and further emitting from a skin (e.g., components and an amount) may not be altered or controlled by own intention. Further, a skin gas including various components with various concentrations is always emitted from various portions on a skin surface of a living body, depending on a physical condition and an environmental change. Thus, it is expected that health control can be achieved by measuring, without awareness, a skin gas from a specific desired portion of the skin surface. That is, for example, if such a skin gas measurement device can be installed in a device that can be worn (which is also described as "wearable device," hereinafter) that is to contact skin, which may be represented by a wristwatch, it is expected that health control through continuous measurement and unaware measurement can be achieved by only wearing the above-described device.

However, an emitted amount of a skin gas component that is emitted from a skin surface and that is associated with a physical condition (e.g., acetone, hydrogen, carbon monoxide, methane, hydrogen sulfide, isoprene, trimethylamine, ammonia, methanol, acetaldehyde, ethanol, nitric monoxide, formaldehyde, and nonenal) is an extremely infinitesimal amount that is less than or equal to an order of ng·cm⁻²·min⁻¹, in general. Thus, it is difficult to measure a skin gas component that is emitted from a skin surface as it is. Consequently, a technique has been studied from the past that is for concentrating and measuring a target skin gas component.

For example, Patent Documents 1 and 2 disclose a technique such that a collected skin gas component is concentrated, and after that the concentrated skin gas component is measured by using a gas chromatography device or the like.

Further, a technique has been known such that a skin gas component is adsorbed and concentrated by using a porous material, the concentrated skin gas component is desorbed by heating the porous material, and then the skin gas component is measured. With a porous material, heating and natural cooling can be reversibly repeated. A porous material has been focused on because, in addition to a natural material, various artificially synthesized materials can be inexpensively produced. For example, Patent Document 3 discloses a technique such that a skin gas component is adsorbed and concentrated by a porous material, and the concentrated skin gas component is desorbed and then measured by an ion mobility sensor. Patent Document 4 discloses a technique such that a skin gas component is adsorbed and concentrated by a porous material, the concentrated skin gas component is desorbed and then measured by a gas chromatography device. Further, Patent Document 5 discloses a technique such that a skin gas component is adsorbed and concentrated by a porous material, the concentrated skin gas component is desorbed, and it is measured by light having a specific wavelength.

### RELATED ART DOCUMENTS

### [PATENT DOCUMENTS]

[Patent Document 1] Japanese Unexamined Patent Publication No. 2004-53571
[Patent Document 2] Japanese Unexamined Patent Publication No. 2009-69137
[Patent Document 3] WO 2012/056729
[Patent Document 4] Japanese Unexamined Patent Publication No. 2012-194088
[Patent Document 5] Japanese Unexamined Patent Publication No. 2005-147962

### SUMMARY OF THE INVENTION

### [PROBLEM TO BE SOLVED BY THE INVENTION]

An embodiment of the present invention may provide a skin gas measurement device and a skin gas measurement method. Specifically, a skin gas measurement device using a skin gas measurement method according to the embodiment of the present invention allows itself to be installed in a wearable device, and allows a skin gas to be continuously and unconsciously measured safely and securely, thereby providing a skin gas measurement device and a skin gas measurement method with which health control can be implemented.

### [MEANS FOR SOLVING THE PROBLEM]

According to an aspect of the present invention, there is provided a skin gas measurement device including a skin gas collecting unit that includes a skin gas collecting space having an opening that is to be attached to a skin surface, a porous material that is for adsorbing and concentrating a skin gas component that is emitted from the skin surface into the skin gas collecting space and that allows the adsorbed skin gas component to be desorbed at a relatively low temperature, and a heater for heating the porous material; and a skin gas measurement unit for measuring the skin gas component that is desorbed from the heated porous material.

Further, according to another aspect of the present invention, there is provided a skin gas measurement method including a step of introducing, from an opening that is attached to a skin surface into a skin gas collecting space, a skin gas component that is emitted from the skin surface; a step of concentrating the introduced skin gas component by causing the introduced skin gas component to be adsorbed by a porous material; a step of desorbing the skin gas component that is adsorbed by the porous material by heating at a relatively low temperature; and a step of measuring a concentration or an amount of the skin gas component that is desorbed.

### [ADVANTAGE OF THE INVENTION]

According to an embodiment of the present invention, a skin gas component that is adsorbed and concentrated by a porous material can be desorbed at a lower (relatively low) temperature than that of a general condition, and a time period that is required for heating and naturally cooling the porous material can be reduced. Thus, emission of the skin gas in a short measurement interval can be monitored. Further, downsizing of the device and long-duration driving of the device can be facilitated because power consumption for heating the porous material can be suppressed to be low. Furthermore, safety and security of the device can be enhanced.

### BRIEF DESCRIPTION OF THE DRAWINGS

FIG. 1 shows a configuration example illustrating a skin gas measurement device according to an embodiment of the present invention;
FIG. 2 shows a table that summarizes properties of five types of zeolites that are different from SiO₂/Al₂O₃ [mol/mol] in a crystal structure and a pore size and that are used in the embodiment of the present invention;
FIG. 3 shows a graph showing adsorption rates of the five types of zeolites for adsorbing acetone that are measured in the embodiment of the present invention;
FIG. 4 shows a graph showing a desorption rate of the five types of zeolites for desorbing acetone that are measured in the embodiment of the present invention;
FIG. 5 shows a graph showing a result of concentrating skin acetone by a thin film of high silica zeolite 390 HUA that is measured in another embodiment of the present invention; and
FIG. 6 is a diagram showing a skin gas measurement method according to an embodiment of the present invention.

### EMBODIMENTS OF THE INVENTION

Patent Documents 1 and 2 disclose a technique such that a collected skin gas component is concentrated, and after that the concentrated skin gas component is measured by using a gas chromatography device or the like. However, in this technique, a cooling agent, such as liquid nitrogen, is used for concentrating the skin gas component.
In general, long-term preservation of such a cooling agent is difficult because the cooling agent tends to be vaporized. Further, experience and skills may be required for handling the cooling agent because there are risks of frostbite and explosion. Thus, it may not be suitable to apply such a method to a wearable device from a technical perspective and from a safety perspective.

Patent Document 3 discloses a technique such that a skin gas component is adsorbed and concentrated by a porous material, the concentrated skin gas component is desorbed, and it is measured by an ion mobility sensor. Patent Document 4 discloses a technique such that a skin gas component is adsorbed and concentrated by a porous material, the concentrated skin gas component is desorbed, and it is measured by a gas chromatography device. Further, Patent Document 5 discloses a technique such that a skin gas component is adsorbed and concentrated by a porous material, the concentrated skin gas component is desorbed, and it is measured by light having a specific wavelength. However, thus far, including these related art documents, an index and a specific condition have not been disclosed that are for selecting a suitable porous material for concentrating a (specific) skin gas component, and that are for further selecting a suitable desorbing condition (e.g., a desorbing temperature) for desorbing under a desirable condition.

It is generally known that, in many cases, a porous material is used for a filter or the like for exhaust gas. In order to sufficiently eliminate (desorb) the adsorbed and concentrated gas component, the adsorbed and concentrated gas component is usually eliminated under a high temperature condition, such as 500 °C as a sufficiently high temperature. However, under a general condition in which the eliminating temperature is a very high temperature, such as 500 °C, a long time period is required for heating and naturally cooling the porous material. There are significant problems, especially for a case of applying to a wearable device, such that emission of the skin gas in a short measurement interval may not be monitored, that power consumption that is required for heating the porous material is increased, and that safety and security of a user are to be secured and considered.

The followings are results, by the inventors, of intensively studying the problems with the above-described related art.
(i) An index for selecting a porous material (specifically, the fact that it is important to suitably select a type, a hydrophilic/hydrophobic degree, a crystal structure, and a pore size or the like of the porous material) has been successfully found. Here, the porous material can sufficiently adsorb and concentrate a skin gas component to be measured, and, upon the porous material being heated to a permissible temperature, the porous material can cause the adsorbed skin gas component to be sufficiently desorbed, thereby allowing the desorbed skin gas component to be measured.
(ii) Further, by using such an index, a suitable porous material has specifically been found such that a skin gas component can be (selectively) adsorbed in a short time period and the skin gas component is concentrated to a desired extent, and the skin gas component can be sufficiently desorbed by heating for a short time period under a non-high temperature condition, such as approximately less than a half or one third of the desorbing temperature, 500 °C, that has been used in the past as a general condition. Here, the porous material allows the skin gas component to be measured with desired accuracy.

Namely, an embodiment of the present invention relates to a porous material that can sufficiently adsorb and concentrate a (specific) skin gas component in a suitable time period; that can cause the skin gas component to be sufficiently desorbed in a suitable time period even if a heating temperature is sufficiently lower than the temperature that has been used as a general condition and the heating temperature meets the user's safety and security; and that allows the subsequent measurement to be made. Further, the embodiment of the present invention relates to a skin gas measurement device or a skin gas measurement method that uses such a porous material.

Namely, a skin gas measurement device according to an embodiment of the present invention is a skin gas measurement device including a skin gas collecting unit that includes a skin gas collecting space having an opening that is to be attached to a skin surface, a porous material that is for adsorbing and concentrating a skin gas component that is emitted from the skin surface into the skin gas collecting space and that allows the adsorbed skin gas component to be desorbed at a relatively low temperature, and a heater for heating the porous material; and a skin gas measurement unit for measuring the skin gas component that is desorbed from the heated porous material.

Here, in the embodiment of the present invention, "the relatively low temperature" may mean a temperature that is lower than a heating temperature that has been used, for the porous material, in the past for desorbing the adsorbed gas component, as specifically explained in detail below.

Further, the skin gas measurement device according to the embodiment of the present invention may be the skin gas measurement device characterized in that the porous material has high hydrophobicity.

Further, the skin gas measurement device according to the embodiment of the present invention may be the skin gas measurement device such that a pore size of the porous material is larger than or equal to a molecular size of the skin gas component and less than or equal to three times the molecular size of the skin gas component.

Further, the skin gas measurement device according to the embodiment of the present invention may be the skin gas measurement device such that the porous material is a zeolite.

Further, the skin gas measurement device according to the embodiment of the present invention may be the skin gas measurement device such that a value of SiO₂/Al₂O₃ [mol/mol] of the zeolite is greater than or equal to 10.

Further, the skin gas measurement device according to the embodiment of the present invention may be the skin gas measurement device such that the skin gas component is acetone or a molecule having a molecular size that is equal to that of acetone.

Further, the skin gas measurement device according to the embodiment of the present invention may be the skin gas measurement device such that the porous material is a zeolite, a pore size of the zeolite is larger than or equal to a molecular size of the skin gas component and less than or equal to three times the molecular size of the skin gas component, and a value of SiO₂/Al₂O₃ [mol/mol] of the zeolite is greater than or equal to 100.

Additionally, a skin gas measurement method according to the embodiment of the present invention is a skin gas measurement method including a step of introducing, from an opening that is attached to a skin surface into a skin gas collecting space, a skin gas component that is emitted from the skin surface; a step of concentrating the introduced skin gas component by causing the introduced skin gas component to be adsorbed by a porous material; a step of desorbing the skin gas component that is adsorbed by the porous material by heating at a relatively low temperature; and a step of measuring a concentration or an amount of the skin gas component that is desorbed.

Here, "the relatively low temperature" may have the same meaning as that of the above-defined "the relatively low temperature." The embodiment of the present invention is explained by referring to the figures.

### (Skin gas measurement device)

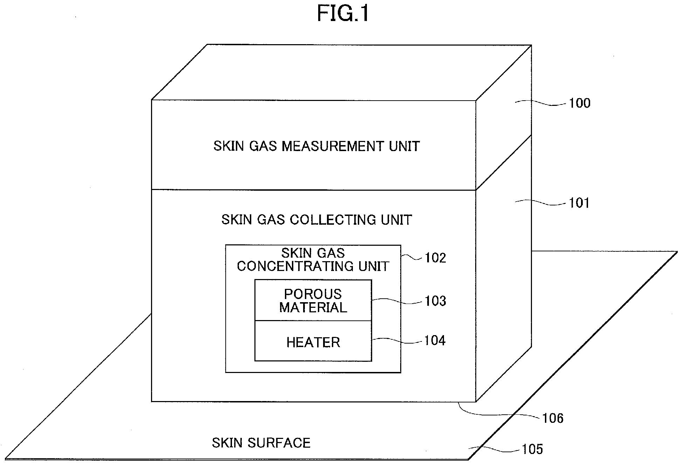

FIG. 1 shows a configuration example of the skin gas measurement device according to the embodiment of the present invention. The skin gas measurement device according to the embodiment of the present invention may include a skin gas measurement unit 100; a skin gas collecting unit 101; a skin gas concentrating unit 102; a porous material 103; a heater 104; and an opening 106.

First, a skin gas component that is emitted from a skin surface 105 is collected for a constant time period from an opening 106 that is attached to the skin surface by the skin gas collecting unit 101. The collected skin gas component is adsorbed by the porous material 103 of the skin gas concentrating unit 102 that exists inside the skin gas collecting unit 101, and the collected skin gas is concentrated. After that, the skin gas component is desorbed by heating the porous material 103 that adsorbs the skin gas component by the heater 104. Subsequently, the desorbed skin gas component is introduced into the skin gas measurement unit 100, and an amount of the skin gas component or concentration of the skin gas component is measured. Details of these processes are explained below.

A skin gas component that is a target to be measured by the skin gas measurement device according to the embodiment of the present invention may include all the skin gas components that are emitted from the skin surface. The skin gas components that are to be measured by the skin gas measurement device according to the embodiment of the present invention especially include skin gas components that are associated with a physical condition (e.g., healthy, illness, sleep, rest, or exercise), and an inorganic component (e.g., water, hydrogen, ammonia, carbon monoxide, carbon dioxide, nitric monoxide, or hydrogen sulfide) and various organic components (e.g., acetone, methanol, ethanol, methane, isoprene, trimethylamine, formaldehyde, acetaldehyde, and nonenal) may be included. Especially, an organic component may have not only its molecular size, but also complex chemical and physical characteristics, such as a molecular polarity or a property of a substituent group. Further, a skin gas component that is emitted from the skin surface and that is a target of the skin gas measurement device according to the embodiment of the present invention may include not only a gas component that is originated from inside the living body, but also a gas component that is originated from outside the living body (originated from an environment). For example, skin gas components can be considered that are emitted from the skin surface after various artificial gas components that are included in a working environment and a living environment are inhaled and absorbed through the skin. Specifically, it has been known that, after being exposed to an organic solvent (e.g., benzene or toluene) or a chlorine-based solvent in an office or a laboratory where chemicals are to be handled, these components are emitted from the skin surface.

The skin gas collecting unit 101 includes the opening 106. The opening 106 can form a closed space when it is attached to the skin surface 105, thereby allowing the skin gas component that is emitted from the skin surface 105 to stay inside the skin gas collecting unit 101. Here, the shape and the size (the area) of the opening 106 are not particularly limited, and the shape and the size that are to be attached to the skin surface 105 so as to form the closed space can be properly selected, depending on the shape and the size of a portion of the skin at which the skin gas that is to be measured is collected. Further, for a case in which it is used for a wearable device, a selection can be made so that it matches the shape and the size of the wearable device. The material of the opening 106 is not particularly limited. However, since the opening 106 is to be attached to the skin, it is desirable that the opening 106 can be formed of a material having favorable adaptability and affinity with respect to a living body, such as hydroxyapatite, silicone, or ceramics. Further, for a case in which the shape of the skin surface 105 is not a plane shape three-dimensionally, such as convexo-concave, it is desirable that the material of the opening 106 can be a flexible material, so that adhesiveness can be maintained. Optionally, the opening 106 may be a single opening. Alternatively, a plurality of the openings 106 may be formed by using some partitions. The opening 106 allows the skin gas to be effectively collected from the skin surface.

Further, it is desirable that the skin gas collecting unit 101 can be formed of a material that does not adsorb the skin gas component that is emitted from the skin surface 105 and that does not emit a gas component that can affect the measurement of the skin gas component. Alternatively, it is desirable that an inner wall of the skin gas collecting unit 101 can be coated with the above-described material. As for these materials, Teflon (registered trademark), glass, a polyvinyl fluoride resin, and so forth can be used, for example. However, the materials are not limited to these.

The skin gas component that stays inside the skin gas collecting unit 101 can be adsorbed by the porous material 103 of the skin gas concentrating unit 102 that exists inside the skin gas collecting unit 101. Here, the porous material that can be used for the embodiment of the present invention is a material that can adsorb the skin gas component and that can cause the skin gas component to be desorbed (removed). Consequently, the porous material that can be used for the embodiment of the present invention is not particularly limited, provided that the material can exhibit such a property. The porous material that can be used for the embodiment of the present invention may include a material that is derived from a natural product, a synthesized material, or a mixture thereof that has been known to have such a property in the past. Additionally, the porous material 103 that can be used for the embodiment of the present invention may include a new porous material that can be newly synthesized based on a guiding principle, which is explained below. Specifically, the porous material 103 according to the embodiment of the present invention may include, for example, a zeolite, porous glass, silica, alumina, activated carbon, molecular sieving carbon, and so forth. However, the porous material 103 is not limited to these.

The porous material is a material having many pores, as it is indicated by the name. For example, a zeolite has a regular porous body having a crystal structure such that four oxygen molecules are regularly and three-dimensionally connected around silicon and aluminum. A zeolite has nanoscale pores whose approximate sizes can be determined by the crystal structure. A molecule that can pass through the pore can be adsorbed inside the porous material. Consequently, "a molecular sieving effect" can be exhibited such that a molecule having a molecular size that is larger than the pore may not be adsorbed. When the size of the pore is properly selected, the porous material according to the embodiment of the present invention can selectively adsorb and concentrate the target skin gas component by the "molecular sieving effect." The crystal structure and the size of the pore of the porous material can be actually measured or estimated by various methods.

However, the inventors have found that, for achieving favorable adsorption and desorption of the target molecule, it may not suffice to select a porous material only by suitability with respect to adsorption by a crystal structure and a pore size. Namely, as shown by an example that is described below, for a case in which the pore size and the molecular size of the target molecule are close, the target molecule can be adsorbed too strongly, and energy that is required for desorbing the target molecule becomes too large. Consequently, a problem may occur such that the temperature for causing the desorption (desorption temperature), which is described below, becomes too high, or a time period for heating for the desorption becomes very long. In contrast, if the pore size is too large compared to the molecular size of the target molecule, unnecessary molecules other than the target molecule can be adsorbed and concentrated, so that selectivity for selecting the target molecule may be lost. Thus, a problem may occur such that measurement of the target skin gas component may be adversely affected. Consequently, it is desirable that the crystal structure and the pore size of the porous material that can be preferably used in the embodiment of the present invention can be such that the pore size is larger than or equal to the molecular size of the molecule that is the target of concentration and less than or equal to several times the molecular size, and especially less than or equal to three times the molecular size. By selecting (or designing) a porous material having a pore size in a preferable range by using this guiding principle, the target skin gas component can be quickly (and selectively) adsorbed and concentrated, and the skin gas component can be quickly desorbed at a desirable desorption temperature, which is explained below.

Further, as shown by the example that is explained below, the inventors have found that, in the embodiment of the present invention, not only the selection by the crystal structure and the pore size, but also hydrophilicity/hydrophobicity (hydrophilicity, hydrophobicity) of the porous material in combination therewith is important as another guiding principle for selecting the porous material. Namely, it has been found that, even if the pore size is almost the same (i.e., even if the pore size is almost equal to the molecular size of the target molecule), by controlling the hydrophilicity/hydrophobicity of the porous material, desorptivity with respect to the target molecule can be significantly improved. For example, when a zeolite is used as a porous material according to the embodiment of the invention, by changing a content ratio between SiO₂ and Al₂O₃, the hydrophilicity/hydrophobicity can be controlled. In general, the hydrophilicity/hydrophobicity of the zeolite can be evaluated by a value of SiO₂/Al₂O₃ [mol/mol]. The smaller this value is, the more hydrophilic the zeolite becomes. The greater this value is, the more hydrophobic the zeolite becomes. The hydrophilicity/hydrophobicity of the porous material according to the embodiment of the present invention can be properly designed and/or selected depending on various calculations based on the molecular structure of the target molecule that is to be adsorbed or actual measurement values, for example. As shown by the example that is described below, in general, for a skin gas component (e.g., an organic molecule, such as acetone or ethanol), even if the pore size is almost the same, a temperature at which the adsorbed target molecule can be desorbed (desorption temperature) becomes lower as the value of SiO₂/Al₂O₃ [mol/mol] becomes greater. Consequently, for quickly desorbing the adsorbed skin gas component at a lower temperature, it is desirable that the hydrophobicity of the porous material can be high. Note that, it is also desirable, from the perspective that the skin gas component is adsorbed without adsorbing moisture, that the hydrophobicity of the porous material is high because a large amount of perspiration and moisture is emitted from the skin surface. Specifically, for zeolite as the porous material that is preferably used in the embodiment of the present invention, it is desirable that the value of SiO₂/Al₂O₃ [mol/mol] can be at least greater than or equal to 10, and can preferably be greater than or equal to 100, for example. By selecting (or designing) a porous material having hydrophilicty/hydrophobicity within the preferable range by using this guiding principle, even if the target skin gas component is a skin gas component having a molecular size that is almost equal to the pore size, the skin gas component can be quickly desorbed at a lower desorption temperature.

As described thus far, for the porous material that is preferably used in the embodiment of the present invention, by properly selecting and/or designing the crystal structure, the pore size, and the hydrophilicity/hydrophobicity, a material can be selected and/or designed that satisfies not only the desired adsorptivity with respect to the target molecule to be adsorbed, but also the desired desorptivity, at the same time. By considering the above-explained guiding principle based on above, for example, for a case in which a skin gas component that is to be concentrated is acetone (the molecular size is 4.6 Å), the high silica zeolite 390 HUA that is produced by Tosoh Corporation, as one of commercial products, can be selected as one of desirable porous materials 103. It has a Y-type crystal structure with a pore size of 7.4Å (which is approximately 1.6 times as large as the molecular size of an acetone molecule), and the value of SiO₂/Al₂O₃ [mol/mol] is 500. The following specifically shows the example in which a porous material that is selected in this manner is used.

Note that it is not necessary that there is one type of the porous material 103 in the skin gas collecting unit 101. Multiple types of the porous materials 103 can be included while they are mixed or separated, depending on the number and types of the skin gas components to be measured. In this manner, simultaneous measurement, stepwise measurement, higher sensitivity measurement, and so forth can be made for a plurality of skin gas components.

Optionally, a moisture removal and suppression system for removing or suppressing moisture that is generated from the skin surface 105, such as water vapor or perspiration, may be included in the skin gas collecting unit 101. Specifically, it can be formed of some of or a combination of an absorbent, a dehumidification agent, a drying agent, and a small blowing device, for example. As for an absorbent, a dehumidification agent, and a drying agent, for example, an A-type zeolite, sodium polyacrylate, silica gel, calcium oxide, calcium chloride, activated carbon, a piece of paper, a fiber, and so forth can be used. However, the absorbent, the dehumidification agent, and a drying agent are not limited to these. Further, for example, by applying air to the skin surface by a small blowing device, the skin surface can be dried, and a condition can be made such that moisture may not be easily generated. In this manner, moisture that may affect the measurement of the target molecule can be removed.

Further, in the embodiment of the present invention, a time period for adsorbing the skin gas component by the porous material and a time period for desorbing the skin gas component are not particularly limited. However, the time periods can be properly adjusted, depending on accuracy of the measurement or an interval of the measurement (or monitoring) by the skin gas measurement device according to the embodiment of the present invention. In order to be installed in a wearable device, and to allow the skin gas component to be continuously and unconsciously measured, so that health control can be implemented, it is desirable that the time periods can be adjusted in a range from few minutes to few hours, respectively.

Further, in the embodiment of the present invention, a concentration factor (an extent of concentration) at the skin gas concentrating unit 102 is not particularly limited. However, in view of sensitivity and a time period of the subsequent measurement by the skin gas measurement unit 100, a concentration factor can preferably be from at least 5 times to approximately 100 times.

Further, the shape, the size, and a quantity of the porous material 103 of the skin gas concentrating unit 102 are not particularly limited. It suffices if there are a shape, a size, and a quantity with which the skin gas component that is collected in the skin gas collecting unit 100 can be sufficiently adsorbed in the above-explained desired time period, and can be sufficiently desorbed in the desired time period. The form of the porous material can be, for example, a powder form, a particle form, a suspension, a dispersion product, a molded form, or the like. When it is used in the powder form or the particle form, it can be placed in a container having a proper shape, such as a column-like shape, and the skin gas component in the collecting space can be adsorbed. Further, when it is used in the form of a suspension or a dispersion product, it can be used by being suspended or dispersed in a proper medium (a liquid or a solid). Further, using it in a molded form may include using a molded body that is obtained by molding the porous material in a specific shape by using an additive, such as a suitable binder. Alternatively, using it in a molded form may include, after further molding, using it as a molded body that is obtained by applying a baking treatment to the molded product. Such a molded product may be desirable because the property as a porous material is maintained, and mechanical strength and thermal strength can be reinforced. In the embodiment of the present invention, it is desirable that the skin gas concentrating unit 102 can be formed to be a thin film shape including the porous material 103. This is made possible by utilizing the related art. For example, for a case of using the above-explained desirable zeolite as the porous material, a thin film of the porous material can be formed on a substrate by slurrying the zeolite with a suitable binder, by thinning the zeolite on the substrate by a suitable means, and by baking it. In the embodiment of the present invention, a substrate that can preferably be used for thinning is not limited. However, glass, ceramics, and metal in various shapes can be considered, for example. Specifically, a silicon wafer can be considered. Further, a binder that can preferably be used for thinning in the embodiment of the present invention is not limited. However, various organic materials, inorganic materials, or mixtures thereof can be considered that are used for forming inorganic powder slurry, for example. Specifically, colloidal silica can be considered as an inorganic material, and various types of modified cellulose can be considered as an organic material. Further, a size of the thin film (thickness, length and width) and selection of the substrate (mechanical characteristics such as a material and flexibility) can be appropriately set depending on a purpose of use. For example, the thickness of the thin film can be in a range from a few µm to several millimeters. For a case of applying the skin gas measurement device according to the embodiment of the present invention to a wearable device, the porous material can preferably be thinned and used, so as to allow downsizing and weight reduction of the wearable device. Additionally, the binder may include an additive for adding various other functions. For example, an additive for improving heat resistance, water resistance, and mechanical stress can be considered.

Further, in order to cause the skin gas component to be desorbed from the porous material 103 by which the skin gas is adsorbed, the heater 104 for heating at a relatively low temperature is included in the skin gas collecting unit 101. Here, a relatively low temperature means a temperature that is lower than a temperature that is usually applied for desorbing the adsorbed component from the porous material. Specifically, it has been found by the inventors that it suffices if the heating temperature for the porous material that is selected by using the above-explained guiding principle is approximately a half or one third of the temperature that has been usually applied in the past for desorbing the adsorbed component. For example, in general, for general-purpose use of a zeolite, the desorption temperature is approximately 500 °C. However, compared to this, for the porous material according to the embodiment of the present invention that is selected by the guiding principle, it has been found by the inventors that the heating temperature for desorbing the target skin gas can be less than 250 °C, or it suffices if it is less than or equal to 200 °C. For desorbing at a high desorption rate, the upper limit of the above-described range is preferable because, as the desorption temperature becomes higher, the desorption rate is increased and the time period that is required for desorption is reduced. However, it is to be considered that the maximum allowable heating temperature can be a temperature such that installation in a wearable device can be assumed, and a user can safely and securely measure the skin gas continuously and unconsciously. In consideration of these points, the upper limit of the heating temperature can preferably be approximately 250 °C. Further, an adsorption/desorption equilibrium of each of the skin gas components can actually be measured. Based on this, a temperature can be estimated at which a desired desorption rate can be obtained. In this manner, the upper limit of the heating temperature can preferably be optimized. By doing this, a skin gas measurement device can be obtained that is safe and secure for the user.

By further considering the safety and security of the user, the device can preferably be designed so that the porous material 103 and the heater 104 may not contact the skin, or may not affect the skin. For example, the skin gas concentrating unit 102 can preferably be disposed at a position that is separated from the skin surface 105. Alternatively, a heat shield, a heat insulating material, and/or a thermal shield may be disposed at a part of or all of the skin gas concentrating unit 102 to the extent that adsorption and desorption of the skin gas component are not affected. As a heat shield and/or a heat insulating material, for example, an aluminum foil, a steel sheet, glass wool, foam glass, foam rubber, and so forth can be used. However, the heat shield and the heat insulating material are not limited to these.

A heating unit of the heater 104 is not particularly limited, provided that the heating unit can heat the porous material 103 to a desired temperature. For example, a ceramic heater, an electric resistance heater, and heating by irradiation of microwaves are available. Further, when the porous material 103 is formed to be a thin film, the electric resistance heater can be provided on the surface of the porous material or inside the porous material by printing or micro processing the electric resistance heater on the thin film of the porous material. In general, many porous materials, such as zeolites, have low thermal conductivity. Thus, for effectively conducting heat from the heater 104 toward inside the porous material 103, the porous material 103 can preferably be used together with a material having high thermal conductivity. A metal material, a carbon material, a ceramic material or the like can be considered as a material having high thermal conductivity. Specifically, it includes sandwiching or embedding the porous material in these materials having high thermal conductivity, or using the porous material while mixing with powder or particles of the materials having high thermal conductivity. By doing these, heating at the heater 104 can be quickly conducted to the porous material 103, thereby achieving effective desorption. It is preferable to apply the skin gas measurement device according to the embodiment of the present invention to a wearable device by doing these because energy saving, downsizing, and weight reduction of the wearable device can be achieved.

The skin gas measurement device according to the embodiment of the present invention further includes the skin gas measurement unit 100. The skin gas measurement unit 100 is for measuring the skin gas component that is heated and desorbed from the porous material 103. Methods and units for introducing the skin gas component that is heated and desorbed from the porous material 103 to the skin gas measurement unit 100 are not particularly limited. It suffices if at least a portion of the skin gas component that is heated and desorbed from the porous material can be introduced to the skin gas measurement unit 100 by the methods and units. For example, by providing a proper closed space between the porous material 103 and the skin gas measurement unit 100, the skin gas component that is heated and desorbed from the porous material 103 can be maintained inside the closed space, and the skin gas component can be measured by the skin gas measurement unit 100. Alternatively, at least a portion of the skin gas component that is desorbed by heating the porous material can be collected by a proper introducing unit (e.g., a tube), and can be introduced to the skin gas measurement unit 100.

The skin gas measurement unit 100 includes a detector (sensor) that is for detecting the target skin gas component that is introduced to the skin gas measurement unit 100, and that is for measuring its amount or concentration. Such a detector is not particularly limited, provided that it is a device that can detect the target skin gas component that is introduced to the skin gas measurement unit 100, and that can measure its amount or concentration. Taking into consideration that the skin gas may include extremely many types of components, and the characteristics of the application of the skin gas measurement device according to the embodiment of the invention (emission of the skin gas in a short measurement interval can be monitored, power consumption can be low, and the device can be easily downsized and driven for a long time period), a semiconductor gas sensor can preferably be used. Specifically, a semiconductor gas sensor can be included that is for measuring various types of gas components that are emitted from a living body, such as acetone, hydrogen, carbon monoxide, methane, hydrogen sulfide, isoprene, trimethylamine, ammonia, methanol, acetaldehyde, ethanol, nitric monoxide, formaldehyde, nonenal, and the like, which are the skin gas components that are concentrated in the porous material 103. Note that the target skin gas components are not limited to the above-described skin gas components. It suffices if a skin gas component is emitted from the skin surface of the living body. Further, the skin gas measurement unit 100 is not limited to the semiconductor gas sensor, and it can be a carbon nanotube type sensor, a graphene type sensor, an electrochemical sensor, an optical fiber sensor, a thin film sensor, an MEMS thermal conductivity sensor, a surface acoustic wave sensor, a micro thermoelectric sensor, a contact combustion sensor, an electromotive force variation type sensor, an optical sensor, or the like. It can be a proper sensor that can measure the skin gas components. Further, the skin gas measurement unit 100 may be a sensor that is for measuring only a specific single gas component. Alternatively, the skin gas measurement unit 100 can be configured such that multiple types of sensors are arranged in an array, so that multiple different skin gas components can be measured. In this case, the skin gas measurement unit 100 is not limited to the arrangement in the array. Further, a downsized gas chromatography chip or the like can be used, so that multiple skin gas components can be measured. Note that the skin gas measurement unit 100 may be disposed inside the skin gas collecting unit 101.

Further, the skin gas measurement unit 100 can preferably include a unit for determining and calculating, which is for determining an amount or concentration of the detected component. For example, a memory for storing information and a processor for calculation can be included. These can be provided inside the skin gas measurement unit 100. Alternatively, these can be separately provided outside the skin gas measurement unit 100. Furthermore, as explained below, these can be provided to a device in which the skin gas measurement device according to the embodiment of the present invention can be installed.

### (Skin gas measurement method)

A skin gas measurement method according to the embodiment of the present invention includes a step of introducing, from an opening that is attached to a skin surface into a skin gas collecting space, a skin gas component that is emitted from the skin surface; a step of concentrating the introduced skin gas component by causing the introduced skin gas component to be adsorbed by a porous material; a step of desorbing the skin gas component that is adsorbed by the porous material by heating at a relatively low temperature; and a step of measuring a concentration or an amount of the skin gas component that is desorbed.

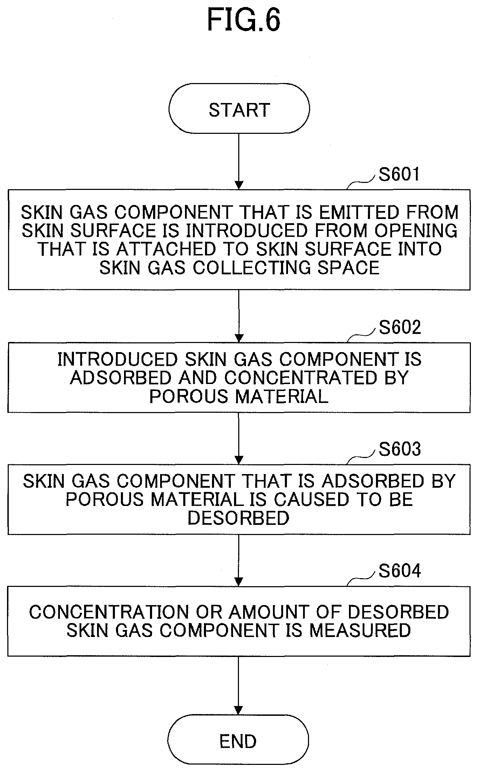

The skin gas measurement method according to the embodiment of the present invention is explained based on FIG. 6. At step S601 of FIG. 6, a skin gas that is emitted from the skin is collected inside the skin gas collecting space, from the opening that is attached to the skin surface into the formed closed space. At step S602, the collected skin gas component is adsorbed by a porous material. Here, as the porous material, the porous material that can preferably be used for the above-explained skin gas measurement device according to the embodiment of the present invention can preferably be used. At step S603, the skin gas component that is adsorbed by the porous material is caused to be desorbed by heating the porous material. Further, at step S604, the desorbed skin gas component is measured. Here, as for the porous material, the heater, the heating temperature, and the measurement unit that can be used for the skin gas measurement method according to the embodiment of the present invention, a reference may be made to the above-described detailed explanation.

By the skin gas measurement method according to the embodiment of the present invention, a user can safely and securely measure the skin gas component continuously and unconsciously.

### (Wearable device including the skin gas measurement device)

By installing the skin gas measurement device according to the embodiment of the present invention in each of various wearable devices, even if the device is always carried, the skin gas can be continuously and unconsciously measured safely and securely, thereby implementing health control.

For this purpose, the skin gas measurement device according to the embodiment of the present invention may include a display unit. Contents to be displayed on the display unit are not particularly limited. However, measurement condition data, such as user data, date and time of measurement, a temperature, and humidity, and a measurement result of the skin gas component can preferably be displayed, for example. By the display, a user can always observe and monitor changes in the skin gas component safely and securely.

Additionally, the skin gas measurement device according to the embodiment of the present invention may further include a communication unit. The communication unit includes a means of communication that is for transmitting and receiving data via a wired line or a wireless channel. The communication unit allows measurement condition data, such as date and time of measurement, a temperature, and humidity; and a measurement result of the skin gas component to be transmitted and received. In this manner, the result can be transmitted and received via online, if desired, between a user and a physician, a nurse, a care person, an exercise instructor, or the like who monitors the user. Thus, a remote medical examination, a remote medical treatment, a remote instruction or the like can be provided to the user. Additionally, by accumulating and managing the data, a remote communication service can be provided, such as providing health advice or exercise guidelines to the user.

Further, a device in which the skin gas measurement device according to the embodiment of the present invention is to be installed can preferably be configured to be a portable type, so that a user can use it any time when needed. For example, a wristwatch-type (wrist), an anklet type (ankle), an adhesive plaster type (back, abdomen, face, etc.), a ring type (finger), a necklace type (the back of the neck), an eyeglass type (temple, near the ear), an earphone/headphone type (near the ear), a shoe type (foot) and the like are possible. Additionally, a portable skin gas measurement device according to the embodiment of the present invention can be configured so that it can be connected to a mobile terminal, such as a cellular telephone, or a personal computer via a wired line or a wireless channel. In this case, the measurement result or the like can preferably be processed, stored, and/or displayed by the mobile terminal or the personal computer. Furthermore, the function of the above-explained communication unit can preferably be implemented in the mobile terminal or the personal computer.

Hereinafter, the embodiment of the present invention is further explained.

### (Example 1)

In this example, a zeolite that is particularly desired to be used is considered as an example. The zeolite is a typical porous material. A result of an experiment is shown that is for demonstrating a principle such that, even if amounts of acetone that are adsorbed by porous materials are the same, amounts of acetone that are desorbed during heating can be different due to differences in the hydrophilic/hydrophobic degree and in the pore size.

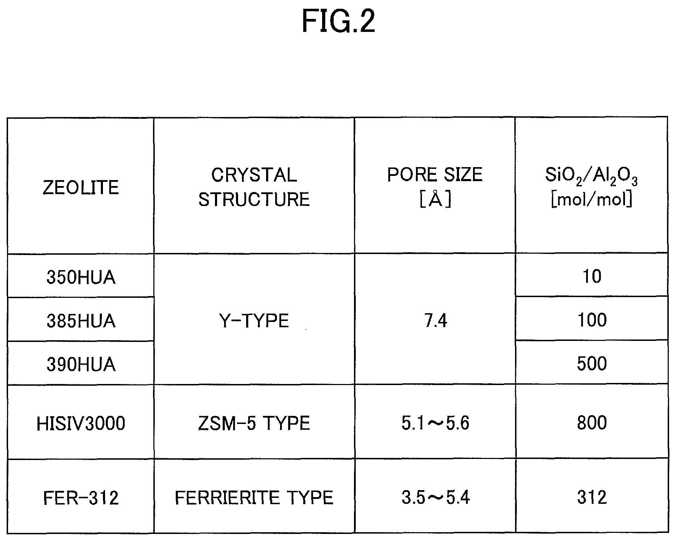

The following five types (FIG. 2) of zeolites were evaluated in this example as an example of porous materials:
high silica zeolites 350HUA, 385HUA, and 390HUA, which are produced by Tosoh Corporation, such that the crystal structures are the same Y-type, but the hydrophilic/hydrophobic degrees are different;
HISIV3000, which is produced by UNION SHOWA K.K., such that the crystal structure is the ZSM-5 type; and
FER-312 (Patent Document 6: Japanese Patent No. 3572365), which is a ferrierite type and chemically synthesized at Okubo Laboratory, Department of Chemical System Engineering, School of Engineering, The University of Tokyo.

Note that the pore size of the Y-type is 7.4Å, the pore size of the ZSM-5 type is 5.1 - 5.6Å, the pore size of the ferrierite type is 3.5 - 5.4Å (Non Patent Document 1: The Structure Commission of the International Zeolite Association, "Database of Zeolite Structures" http://www.iza-structure.org/databases), and the molecular size of acetone is 4.6Å.

### [Preprocessing/Adsorption]

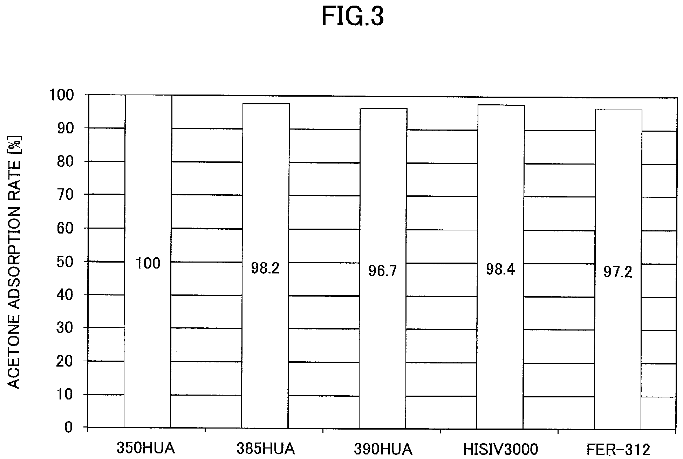

Powder (5 mg) of each of the zeolites from which unnecessary gas components including acetone were desorbed in advance was put inside a sealed vial (volume was 16.9 mL) having a septum through which gas could be taken in and out. A pure acetone gas (the amount of acetone was 40.8 ng) that was diluted with nitrogen was injected, and it was left for fifteen minutes. After that, an amount of acetone included in the atmosphere inside the vial was measured by a gas chromatography device, and an acetone adsorption rate for the powder of each of the zeolites was calculated. FIG. 3 is the result.

From FIG. 3, it can be seen that almost no amount of acetone was detected in the atmosphere inside the vial, and that the powder of each of the five types of zeolites could adsorb almost all acetone that was injected.

### [Desorption]

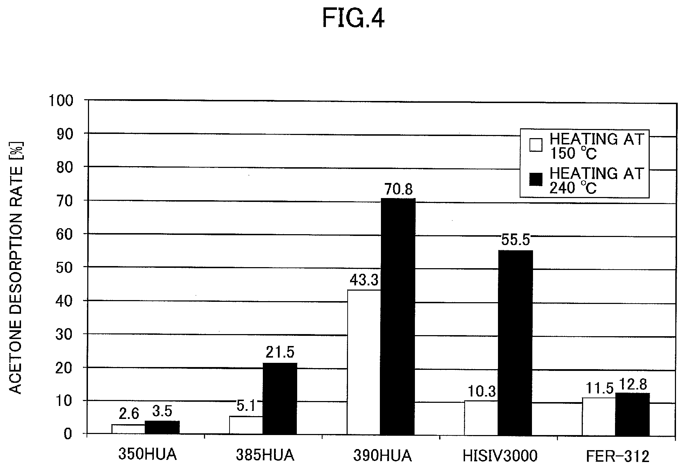

The powder (5 mg) of each of the zeolites by which acetone was adsorbed, as described above, was heated in the sealed vial (volume was 16.9 mL) having the septum through which a gas could be taken in and out for five minutes under a non-high temperature condition (150 °C or 240 °C), such as one third or a half of the heating temperature of the past, and thereby acetone was desorbed. After that, an amount of acetone included in the atmosphere inside the vial was measured by the gas chromatography device, and an acetone desorption rate for the powder of each of the zeolites was calculated. FIG. 4 is the result.

From FIG. 4, it can be seen that, for each of the five types of zeolites, the acetone desorption rate was the greater for heating at 240 °C; and the acetone desorption rate became greater, as the heating temperature was increased. Though it is not shown in the figure, the acetone desorption rate that is close to 100% can be obtained if the heating temperature is greater than or equal to 500 °C.

Subsequently, from the comparison among 350HUA, 385HUA, and 390HUA that have the same crystal structures, it can be seen that the greater the hydrophobicity was, the greater the acetone desorption rate became. Thus, for desorbing at a lower temperature, it is desirable that the hydrophobicity is greater. Additionally, from the comparison among 390HUA, HISIV3000, and FER-312 that have different pore sizes, it can be seen that the larger the pore size of the zeolite was, the greater the acetone desorption rate became. Thus, for desorbing at a lower temperature, it is desirable that the pore size of the zeolite is larger.

### (Example 2)

In the example 1, the results of evaluating basic performance were shown for the case of adsorbing and desorbing the pure gas of acetone. However, it is necessary to show that acetone that is emitted from an actual human skin surface (which is referred to as skin acetone, hereinafter) can be similarly adsorbed and desorbed by a porous material.

Thus, in this example, a sample that was obtained by thinning the high silica zeolite 390HUA (which is referred to as the 390HUA thin film, hereinafter), which was produced by Tosoh Corporation, was used as an example of the porous material. An experiment was conducted that was for demonstrating that the skin acetone was adsorbed by the 390HUA thin film by changing the time period for collecting the skin gas component, and after that the skin acetone was desorbed.

### [Production of 390HUA thin film]

Slurry was produced by adding 4.42 g of SNOWTEX (NISSAN CHEMICAL INDUSTRIES. LTD.) and 0.35 g of carboxymethyl-cellulose (Wako Pure Chemical Industries, Ltd.), and 20 mL of super pure water to 4.42 g of powder of 390HUA, and by sufficiently mixing it.

The produced slurry was dropped onto a silicone wafer of 8 mm × 8 mm square, and it was spin coated for 30 seconds at 2000 rpm. After that, the moisture that was included in the slurry was evaporated by heating at 100 °C for 30 minutes by an electric furnace. Then, the 390HUA thin film was produced by baking for 60 minutes at 800 °C. The amount of the zeolite that was included in the 390HUA thin film that was produced by this method was approximately 4 mg.

### [Adsorption/Desorption of skin acetone]

The produced 390HUA thin film was put into a vial with its lid opened (the area of the opening was 1.13 cm², the volume was 16.9 mL). The opening of the vial was attached to the skin surface of the examinee, and the skin gas component was collected for 5 minutes, 15 minutes, or 30 minutes. Note that, even if the opening of the vial was separated, e.g., by body movement of the examinee, from the skin surface during the time period of the collection, and the hermeticity of the skin gas collecting space was temporarily broken, it could not be a significant obstacle to the measurement because the skin gas component that was adsorbed by the 390HUA thin film might not be desorbed, provided that the skin gas component was not heated.

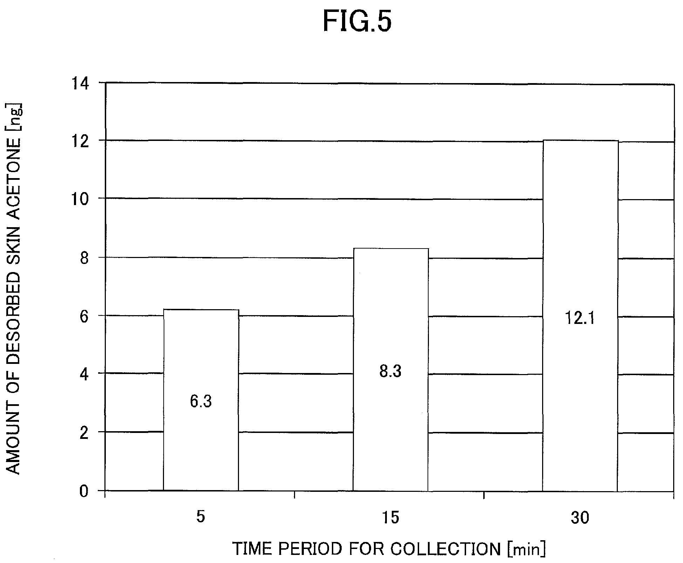

After collection, the 390HUA thin film by which the skin gas component was adsorbed was put into a sealed vial (the volume was 16.9 mL) having a septum through which a gas could be taken in and out, the 390HUA thin film was heated for 5 minutes at 240 °C, and the adsorbed skin gas component was desorbed. After that, an amount of acetone that was included in the atmosphere in the vial was measured by a gas chromatography device, and thereby the amount of acetone that was desorbed from the 390HUA thin film was calculated. FIG. 5 is the result.

From FIG. 5 it can be seen that, as the time period for collection was increased, an amount of the desorbed skin acetone was increased. Thus, by increasing the time period for collection, the amount of the skin acetone that is adsorbed by the 390HUA thin film and that is desorbed from the 390HUA thin film can be increased.

Further, the average value of an amount of skin acetone that was naturally emitted per minute from the skin surface area of 1.13 cm² that was used for the collection was calculated, and it was 0.75 ng. For the case in which the skin gas was collected for five minutes and then the skin gas was desorbed, an amount of acetone that was emitted was 6.3 ng. Thus, compared to the case in which the amount of skin acetone that was naturally emitted was measured in real time without using the porous material, an acetone concentrating effect of 8.4 times was obtained for the collection for 5 minutes. Similarly, an acetone concentrating effect of 11.0 times was obtained for the collection for 15 minutes, and an acetone concentrating effect of 16.1 times was obtained for the collection of 30 minutes.

Note that, from the results of the experiments of the above-described example 1 and example 2, it can be said that, in order to desorb the skin acetone from the zeolite at a temperature that is lower than that of the usual condition, 390HUA can most preferably be used. However, the use is not limited by 390HUA. It can be said that, though the skin acetone concentrating effect is more or less lower, the other zeolites, such as HISIV3000, 385HUA, or FER-312, have sufficient potential for use, depending on the time period for collecting the skin gas and performance of a sensor in the skin gas measurement unit 100, such as the detection limit. In contrast, the skin acetone concentrating effect may hardly be obtained by 350HUA. Thus, 350HUA may not be suitable for use.

By the above results, it is shown that by properly selecting the type, the hydrophilic/hydrophobic degree, the crystal structure, the pore size, and the like of the porous material, depending on the type and the molecular size of the skin gas component to be measured, the adsorbed skin gas component can be desorbed from the porous material at a temperature that is lower than that of the usual condition, and the emission of the skin gas component can be monitored in a short measurement interval by a wearable device.

The skin gas measurement device and the skin gas measurement method for measuring the skin gas component by collecting and concentrating the skin gas component are explained above by the embodiment. However, the present invention is not limited to the above-described embodiment, and various modifications and improvements may be made within the scope of the present invention. For convenience of the explanation, specific examples of numerical values are used in order to facilitate understanding of the invention. However, these numerical values are simply illustrative, and any other appropriate values may be used, except as indicated otherwise. For the convenience of explanation, the devices according to the embodiments of the present invention are explained by using functional block diagrams. However, these devices may be implemented in hardware, software, or combinations thereof. The separations of the items in the above explanation are not essential to the present invention. Depending on necessity, subject matter described in two or more items may be combined and used, and subject matter described in an item may be applied to subject matter described in another item (provided that they do not contradict).

The present international application is based on and claims the benefit of priority of Japanese Patent Application No. 2013-113392, filed on May 29, 2013, the entire contents of Japanese Patent Application No. 2013-113392 are hereby incorporated by reference.

### LIST OF REFERENCE SYMBOLS

- 100:: Skin gas measurement unit
- 101:: Skin gas collecting unit
- 102:: Skin gas concentrating unit
- 103:: Porous material
- 104:: Heater
- 105:: Skin surface
- 106:: Opening

## Claims

1. A skin gas measurement device comprising:
a skin gas collecting unit that includes a skin gas collecting space having an opening that is to be attached to a skin surface, a porous material that is for adsorbing and concentrating a skin gas component that is emitted from the skin surface into the skin gas collecting space and that allows the adsorbed skin gas component to be desorbed at a relatively low temperature, and a heater for heating the porous material; and
a skin gas measurement unit for measuring the skin gas component that is desorbed from the heated porous material.

2. The skin gas measurement device according to claim 1,
wherein the porous material is hydrophobic.

3. The skin gas measurement device according to claim 1,
wherein a pore size of the porous material is larger than or equal to a molecular size of the skin gas component and less than or equal to three times the molecular size of the skin gas component.

4. The skin gas measurement device according to claim 1,
wherein the porous material is a zeolite.

5. The skin gas measurement device according to claim 4,
wherein a value of SiO₂/Al₂O₃ [mol/mol] of the zeolite is greater than or equal to 10.

6. The skin gas measurement device according to claim 1,
wherein the skin gas component is acetone or a molecule having a molecular size that is equal to that of acetone.

7. The skin gas measurement device according to claim 1,
wherein the porous material is a zeolite, a pore size of the zeolite is larger than or equal to a molecular size of the skin gas component and less than or equal to three times the molecular size of the skin gas component, and a value of SiO₂/Al₂O₃ [mol/mol] of the zeolite is greater than or equal to 100.

8. A skin gas measurement method comprising:
a step of introducing, from an opening that is attached to a skin surface into a skin gas collecting space, a skin gas component that is emitted from the skin surface;
a step of concentrating the introduced skin gas component by causing the introduced skin gas component to be adsorbed by a porous material;
a step of desorbing the skin gas component that is adsorbed by the porous material by heating at a relatively low temperature; and
a step of measuring a concentration or an amount of the skin gas component that is desorbed.
